# EUROPÄISCHE PATENTSCHRIFT

(11) **EP 2 140 853 B1**
(45) Veröffentlichungstag und Bekanntmachung des Hinweises auf die Patenterteilung: **18.07.2018**
(21) Anmeldenummer: 09154617.6
(22) Anmeldetag: 09.03.2009
(51) Int. Cl.: A61K 8/11, A61K 8/25, A61K 8/92, A61Q 5/02, A61Q 11/00, A61Q 13/00, A61Q 19/10

(54) **Beschichtete Partikel für den Einsatz in Kosmetika**
Coated particle for use in cosmetics
Particule enrobée pour l'utilisation cosmétique

(30) Priorität: 01.07.2008 DE 102008030662
(43) Veröffentlichungstag der Anmeldung: 06.01.2010
(73) Patentinhaber: Henkel AG & Co. KGaA, 40589 Düsseldorf (DE)
(72) Erfinder: Barth, Adolf Peter, 42781 Haan (DE); Poth, Tilo, 69469 Weinheim (DE); Leinen, Hans-Theo, 40699 Erkrath (DE)

(56) Entgegenhaltungen:
- WO-A1-2004/083356
- WO-A1-2007/128326
- WO-A2-2008/072239

## Beschreibung

Die Erfindung betrifft beschichtete Partikel für den Einsatz in Kosmetika und spezielle kosmetische Zusammensetzungen, die diese Partikel enthalten.

Es ist bekannt, kosmetische Zubereitungen Geruchs- und/oder Geschmacksstoffe bzw. Aromen zuzusetzen, um ihnen einerseits einen besonderen Geruch bzw. Geschmack zu verleihen und andererseits um das Verbraucherinteresse nach einem einzigartigen Produktempfinden zu befriedigen. Es besteht daher stets der Bedarf, dem Verbraucher ein "typisches und unverwechselbares" Produkt zur Verfügung zu stellen, das er wiederholt kauft und anwendet. Zur Vermeidung hygienischer Bedenken werden kosmetische Zubereitungen oft so verpackt, daß ein direkter Kontakt mit dem Produkt am Verkaufsort nicht möglich ist. Dabei weisen die Verpackungen Sicherungsmittel (z.B. peel-of-Folien) auf, die verhindern, daß das Produkt am Verkaufsort Kontakt mit der Umgebung erhält. Der Verbraucher kann auch durch Abschrauben des Verschlusses das Produkt folglich nicht olfaktorisch wahrnehmen. Dies führt dazu, daß es weiterer Anstrengungen bedarf, das Produkt bereits am Verkaufsort als "typisches und unverwechselbares" Produkt darzustellen. Hier bieten sich neben der Verpackungsgestaltung auch optische Differenzierungen des Produkts an.

Der vorliegenden Erfindung lag die Aufgabe zugrunde, Zubereitungsformen für Kosmetika bereitzustellen, die bereits am Verkaufsort ein einzigartiges Empfinden hervorrufen.

Auf dem Gebiet der Wasch- und Reinigungsmittel ist es hierzu seit langem üblich, so genannte "speckles", d.h. Sprenkel einzusetzen, die aus gefärbten Partikeln bestehen. Teilchenförmige Additive zur Beduftung von Waschflotten und zum Einsatz in Wasch- und Reinigungsmitteln sowie Verfahren zu ihrer Herstellung sind beispielsweise in den internationalen Patentanmeldungen WO 97/29176 A1 und WO 97/29177 A1 (Procter & Gamble) beschrieben. Nach der Lehre dieser Schriften werden poröse Trägermaterialien (z. B. Sucrose im Gemisch mit Zeolith X) mit Parfüm versetzt und schließlich mit einem coating-Material (Carbohydrate) überzogen und auf die gewünschte Teilchengrößenverteilung gebracht. Diese Farbsprenkel können in pulverförmigen Mitteln eingesetzt werden, in flüssigen Zubereitungen sind die Probleme der Auslaugung der Partikel ("Ausbluten") noch deutlicher, so daß hier spezielle Maßnahmen ergriffen werden müssen, um stabile optische Differenzierungen zu ermöglichen.

Die WO 2008/072239 A2 offenbart mit einer Silikatschicht umgebene Geruchsstoffe in Form von Nanokapseln.
In der WO 2004/083356 A1 werden feste, unbeschichtete parfümtragende Partikel offenbart, welche in Pulverwaschmittel eingearbeitet werden können und leicht löslich sind.
Der vorliegenden Erfindung lag die Aufgabe zugrunde, eine weitere optische Differenzierungsmöglichkeit zum Einsatz in Kosmetika bereitzustellen, ohne daß es bei Lagerung zum Ausbluten kommt.
Es wurde nun gefunden, daß sich Silikatpartikel mit Silikatbeschichtung zur optischen Differenzierung von Kosmetika eignen und auch in flüssige Kosmetika stabil einarbeitbar sind.
Gegenstand der vorliegenden Erfindung sind in einer ersten Ausführungsform beschichtete Duft- und/oder Aromapartikel, umfassend einen Partikelkern und eine diesen Kern umgebende Hülle, wobei
a) der Partikelkern - bezogen auf sein Gewicht - 80 bis 99,99 Gew.-% Silikat(e) enthält und
b) der Partikelkern - bezogen auf sein Gewicht - 0,01 bis 20 Gew.-% Duftstoff(e) und/oder Aromen enthält und
c) die den Kern umgebende Hülle - bezogen auf ihr Gewicht - 80 bis 100 Gew.-% wasserlösliche Kalium- und/oder Natriumsilikate enthält.
Erfindungsgemäße Partikel enthalten demnach mindestens einen duftstoff- und/oder aromhaltigen Partikelkern, welcher mit einer Hülle umgeben ist.
Der Kern der erfindungsgemäßen beschichteten Partikel enthält - bezogen auf sein Gewicht - 80 bis 99,99 Gew.-% Silikat(e). Silikate sind Salze und Ester der Orthokieselsäure Si(OH)₄ und deren Kondensationsprodukte. Mit besonderem Vorzug wird/werden erfindungsgemäß als Silikat(e) Kieselsäure(n) eingesetzt. Erfindungsgemäß bevorzugt werden *Fällungskieselsäuren* als Partikelkern eingesetzt. Sie werden aus einer wäßrigen Alkalisilicat-Lösung durch Fällung mit Mineralsäuren hergestellt. Dabei bilden sich kolloidale Primärteilchen, die mit fortschreitender Reaktion agglomerieren und schließlich zu Aggregaten verwachsen. Die pulverförmigen, voluminösen Formen besitzen üblicherweise Porenvolumina von 2,5-15 mL/g und spezifische Oberflächen von 30-800 m²/g. Erfindungsgemäß bevorzugte beschichtete Duft- und/oder Aromapartikel, umfassen einen Partikelkern und eine diesen Kern umgebende Hülle, wobei
a) der Partikelkern - bezogen auf sein Gewicht - 80 bis 99,99 Gew.-% Fällungskieselsäure(n) enthält und
b) der Partikelkern - bezogen auf sein Gewicht-0,01 bis 20 Gew.-% Duftstoff(e) und/oder Aromen enthält und
c) die den Kern umgebende Hülle - bezogen auf ihr Gewicht - 80 bis 100 Gew.-% wasserlösliche Kalium- und/oder Natriumsilikate enthält.
Erfindungsgemäße Partikel sind dadurch gekennzeichnet, daß das bzw. die Silikat(e) des Partikelkerns Partikelgrößen zwischen 10 und 1000 µm aufweisen, wobei bevorzugte Silikate eine gewichtsmittlere Partikelgrößenverteilung aufweisen, bei der > 70 Gew.-%, vorzugsweise > 80 Gew.-% und insbesondere > 90 Gew.-% der Teilchen Größen zwischen 100 und 700 µm aufweisen.

Zusätzlich zu Silikat(en) kann der Partikelkern weitere Inhaltsstoffe in Mengen von 0,01 bis 20 Gew.-% enthalten. In diese Gruppe der weiteren Inhaltsstoffe fallen insbesondere die Duftstoff(e) und/oder Aromen als Inhaltstoff b), es ist aber auch möglich, weitere Trägermaterialien einzusetzen. Hier sind erfindungsgemäße Partikel bevorzugt, bei denen der Partikelkern zusätzlich Titandioxid enthält.

Als weitere Inhaltsstoffe des Partikelkerns bieten sich beispielsweise Farbstoffe, Duftstoffe, Öle, Aromen, Antikarieswirkstoffe, Wirkstoffe gegen Halitosis usw. an, wobei der Partikelkern - bezogen auf sein Gewicht - zwingend 0,01 bis 20 Gew.-% Duftstoff(e) und/oder Aromen enthält.

Als Parfümöle bzw. Duftstoffe können erfindungsgemäß einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, α-Isomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl. Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester. Zu den leichter flüchtigen Riechstoffen zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Als wasserunlösliche Aromaöle kommen alle gebräuchlichen natürlichen und synthetischen Aromen infrage. Natürliche Aromen können sowohl in Form der aus den Drogen isolierten ätherischen Öle als auch in Form der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Besonders bevorzugte erfindungsgemäße Partikel sind dadurch gekennzeichnet, daß der Partikelkern - bezogen auf sein Gewicht - 0,05 bis 19 Gew.-%, vorzugsweise 0,1 bis 18 Gew.-%, besonders bevorzugt 0,5 bis 17 Gew.-%, weiter bevorzugt 0,75 bis 16 Gew.-% und insbesondere 1 bis 15 Gew.-% mindestens eines Aromas aus der Gruppe Pfefferminz, Spearmint, Menthol, Limone, Limette, Apfel, Orange, Zitrone, Erdbeere, Aloe Vera, Kaugummi enthalten.

Die beschichteten Partikel können im Partikelkern zusätzlich zu Aromen und/oder Duftstoffen auch scharf schmeckende oder ein Gefühl von Wärme erzeugende Substanzen enthalten. Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise 2E,4E-Decadiensäure-N-Methylamid, 2E,4E-Decadiensäure-N-Ethylamid, 2E,4E-Decadiensäure-N-n-Propylamid, 2E,4E-Decadiensäure-N-Isopropylamid, 2E,4E-Decadiensäure-N-n-Butylamid, 2E,4E-Decadiensäure-N-(1-Methylpropyl)-amid, 2E,4E-Decadiensäure-N-Isobutylamid, 2E,4E-Decadiensäure-N-tert-Butylamid, 2E,4Z-Decadiensäure-N-Methylamid, 2E,4Z-Decadiensäure-N-Ethylamid, 2E,4Z-Decadiensäure-N-n-Propylamid, 2E,4Z-Decadiensäure-N-Isopropylamid, 2E,4Z-Decadiensäure-N-n-Butylamid, 2E,4Z-Decadiensäure-N-(1-Methylpropyl)-amid, 2E,4Z-Decadiensäure-N-Isobutylamid, 2E,4Z-Decadiensäure-N-tert-Butylamid, 2E,4E,8Z-Decatriensäure-N-Methylamid, 2E,4E,8Z-Decatriensäure-N-Ethylamid, 2E,4E,8Z-Decatriensäure-N-n-Propylamid, 2E,4E,8Z-Decatriensäure-N-Isopropylamid, 2E,4E,8Z-Decatriensäure-N-n-Butylamid, 2E,4E,8Z-Decatriensäure-N-(1-Methylpropyl)-amid, 2E,4E,8Z-Decatriensäure-N-Isobutylamid, 2E,4E,8Z-Decatriensäure-N-tert-Butylamid, 2E,4Z,8Z-Decatriensäure-N-Methylamid, 2E,4Z,8Z-Decatriensäure-N-Ethylamid, 2E,4Z,8Z-Decatriensäure-N-n-Propylamid, 2E,4Z,8Z-Decatriensäure-N-Isopropylamid, 2E,4Z,8Z-Decatriensäure-N-n-Butylamid, 2E,4Z,8Z-Decatriensäure-N-(1-Methylpropyl)-amid, 2E,4Z,8Z-Decatriensäure-N-Isobutylamid, 2E,4Z,8Z-Decatriensäure-N-tert-Butylamid, 2E,4E,8E-Decatriensäure-N-Methylamid, 2E,4E,8E-Decatriensäure-N-Ethylamid, 2E,4E,8E-Decatriensäure-N-n-Propylamid, 2E,4E,8E-Decatriensäure-N-Isopropylamid, 2E,4E,8E-Decatriensäure-N-n-Butylamid, 2E,4E,8E-Decatriensäure-N-(1-Methylpropyl)-amid, 2E,4E,8E-Decatriensäure-N-Isobutylamid, 2E,4E,8E-Decatriensäure-N-tert-Butylamid, 2E,4Z,8E-Decatriensäure-N-Methylamid, 2E,4Z,8E-Decatriensäure-N-Ethylamid, 2E,4Z,8E-Decatriensäure-N-n-Propylamid, 2E,4Z,8E-Decatriensäure-N-Isopropylamid, 2E,4Z,8E-Decatriensäure-N-n-Butylamid, 2E,4Z,8E-Decatriensäure-N-(1-Methylpropyl)-amid, 2E,4Z,8E-Decatriensäure-N-Isobutylamid, 2E,4Z,8E-Decatriensäure-N-tert-Butylamid, 2E,6Z,8E-Decatriensäure-N-Methylamid, 2E,6Z,8E-Decatriensäure-N-Ethylamid, 2E,6Z,8E-Decatriensäure-N-n-Propylamid, 2E,6Z,8E-Decatriensäure-N-Isopropylamid, 2E,6Z,8E-Decatriensäure-N-n-Butylamid, 2E,6Z,8E-Decatriensäure-N-(1-Methylpropyl)-amid, 2E,6Z,8E-Decatriensäure-N-Isobutylamid, 2E,6Z,8E-Decatriensäure-N-tert-Butylamid, 2E,6E,8E-Decatriensäure-N-Methylamid, 2E,6E,8E-Decatriensäure-N-Ethylamid, 2E,6E,8E-Decatriensäure-N-n-Propylamid, 2E,6E,8E-Decatriensäure-N-Isopropylamid, 2E,6E,8E-Decatriensäure-N-n-Butylamid, 2E,6E,8E-Decatriensäure-N-(1-Methylpropyl)-amid, 2E,6E,8E-Decatriensäure-N-Isobutylamid, 2E,6E,8E-Decatriensäure-N-tert-Butylamid, 2E,6Z,8Z-Decatriensäure-N-Methylamid, 2E,6Z,8Z-Decatriensäure-N-Ethylamid, 2E,6Z,8Z-Decatriensäure-N-n-Propylamid, 2E,6Z,8Z-Decatriensäure-N-Isopropylamid, 2E,6Z,8Z-Decatriensäure-N-n-Butylamid, 2E,6Z,8Z-Decatriensäure-N-(1-Methylpropyl)-amid, 2E,6Z,8Z-Decatriensäure-N-Isobutylamid, 2E,6Z,8Z-Decatriensäure-N-tert-Butylamid, 2E,6E,8Z-Decatriensäure-N-Methylamid, 2E,6E,8Z-Decatriensäure-N-Ethylamid, 2E,6E,8Z-Decatriensäure-N-n-Propylamid, 2E,6E,8Z-Decatriensäure-N-Isopropylamid, 2E,6E,8Z-Decatriensäure-N-n-Butylamid, 2E,6E,8Z-Decatriensäure-N-(1-Methylpropyl)-amid, 2E,6E,8Z-Decatriensäure-N-Isobutylamid, 2E,6E,8Z-Decatriensäure-N-tert-Butylamid, 2E,7Z,9E-Undecatriensäure -N-Methylamid, 2E,7Z,9E-Undecatriensäure -N-Ethylamid, 2E,7Z,9E-Undecatriensäure -N-n-Propylamid, 2E,7Z,9E-Undecatriensäure -N-Isopropylamid, 2E,7Z,9E-Undecatriensäure -N-n-Butylamid, 2E,7Z,9E-Undecatriensäure -N-(1-Methylpropyl)-amid, 2E,7Z,9E-Undecatriensäure-N-isobutylamid, 2E,7Z,9E-Undecatriensäure -N-tert-Butylamid, 2E,7E,9E-Undecatriensäure -N-Methylamid, 2E,7E,9E-Undecatriensäure -N-Ethylamid, 2E,7E,9E-Undecatriensäure -N-n-Propylamid, 2E,7E,9E-Undecatriensäure -N-Isopropylamid, 2E,7E,9E-Undecatriensäure -N-n-Butylamid, 2E,7E,9E-Undecatriensäure -N-(1-Methylpropyl)-amid, 2E,7E,9E-Undecatriensäure-N-isobutylamid, 2E,7E,9E-Undecatriensäure -N-tert-Butylamid, 2E,7Z,9Z-Undecatriensäure -N-Methylamid, 2E,7Z,9Z-Undecatriensäure -N-Ethylamid, 2E,7Z,9Z-Undecatriensäure -N-n-Propylamid, 2E,7Z,9Z-Undecatriensäure -N-Isopropylamid, 2E,7Z,9Z-Undecatriensäure -N-n-Butylamid, 2E,7Z,9Z-Undecatriensäure -N-(1-Methylpropyl)-amid, 2E,7Z,9Z-Undecatriensäure-N-isobutylamid, 2E,7Z,9Z-Undecatriensäure -N-tert-Butylamid, 2E,7Z,9E-Undecatriensäure -N-Methylamid, 2E,7Z,9E-Undecatriensäure -N-Ethylamid, 2E,7Z,9E-Undecatriensäure -N-n-Propylamid, 2E,7Z,9E-Undecatriensäure -N-Isopropylamid, 2E,7Z,9E-Undecatriensäure -N-n-Butylamid, 2E,7Z,9E-Undecatriensäure -N-(1-Methylpropyl)-amid, 2E,7Z,9E-Undecatriensäure-N-isobutylamid, 2E,7Z,9E-Undecatriensäure -N-tert-Butylamid.
Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..
Unter den genannten Verbindungen sind einige im Rahmen der vorliegenden Erfindung besonders bevorzugt. Diese sind im Prioritätsdokument DE 10 2008 230 662 A1 detaillierter mit Strukturformeln aufgeführt:
2E,6Z,8E-Decatriensäure-N-isobutylamid (N-Isobutyl-2E,6Z,8E-decatrienamid, auch Spilanthol oder Affinin genannt), 2E,4E,8Z-Decatriensäure-N-isobutylamid (N-Isobutyl-2E,4E,8Z-decatrienamid, auch Isoaffinin genannt), 2E,7Z,9E-Undecatriensäure-N-isobutylamid (N-Isobutyl-2E,7Z,9E-undecatrienamid),
2E,4Z-Decadiensäure-N-isobutylamid (cis-Pellitorin), 2E,4E-Decadiensäure-N-isobutylamid (trans-Pellitorin), Ferulasäureamide, beispielsweise Ferulasäure-N-Vanillylamid, *N*-[2-(4-Hydroxy-3-methoxyphenyl)ethyl]-3-(4-hydroxy-3-methoxy-phenyl)-(2*E*)-propensäureamid(trans-Feruloylmethoxytyramin), *N*-[2-(4-Hydroxy-3-methoxyphenyl)ethyl]-3-(4-hydroxy-3-methoxy-phenyl)-(2 *Z*)-propensäureamid (cis-Feruloylmethoxytyramin), *N*-[2-(4-Hydroxy-3-methoxyphenyl)ethyl]-3-(4-hydroxy-3-methoxy-phenyl)-propansäureamid (Dihydroferuloylmethoxytyramin), *N*-[2-(3,4-Dihydroxyphenyl)ethyl]-3-(4-hydroxy-3-methoxy-phenyl)-(2*E*)-propensäureamid (trans-Feruloyldopamin), *N*-[2-(3,4-Dihydroxyphenyl)ethyl]-3-(4-hydroxy-3-methoxy-phenyl)-(2*Z*)-propensäureamid (cis- Feruloyldopamin), *N*-[2-(4-Hydroxyphenyl)ethyl]-3-(3,4-dihydroxyphenyl)-(2*E*)-propensäureamid (trans-Caffeoyltyramin), *N*-[2-(4-Hydroxyphenyl)ethyl]-3-(3,4-dihydroxyphenyl)-(2*Z*)-propensäureamid (cis-Caffeoyltyramin), *N*-[2-(3,4-Dimethoxyphenyl)ethyl]-3-(3,4-dimethoxyphenyl)-(2*E*)-propensäureamid (trans- Rubenamin), *N*-[2-(3,4-dimethoxyphenyl)ethyl]-3-(3,4-dimethoxyphenyl)-(2*Z*)-propensäureamid (cis-Rubenamin).

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*), Wasserpfefferextrakt (*Polygonum ssp.* ,insbesondere *Polygonum hydropiper*), Extrakte aus *Allium* ssp.(insbesondere Zwiebel und Knoblauchextrakte), Extrakte aus Rettich (*Raphanus ssp.*), Meerrettichextrakte (*Cochlearia armoracia*), Extrakte aus schwarzem (*Brassica nigra*), wildem oder gelbem Senf (*Sinapis ssp.,* insbesondere *Sinapis arvensis* und *Sinapis alba*), Bertramwurzel-Extrakte (*Anacyclus ssp.,* insbesondere *Anacyclus pyrethrum*L.), Sonnenhutextrakte (*Echinaceae ssp.*), Extrakten aus Szechuan-Pfeffer (*Zanthoxylum ssp.,* insbesondere *Zanthoxylum piperitum*), Spilanthesextrakt (*Spilanthes ssp.,* insbesondere *Spilanthes acmella*), Chiliextrakt (*Capsicum ssp.,* insbesondere *Capsicum frutescens*), Paradieskörner-Extrakt ( *Aframomum* ssp.,insbesondere *Aframomum melegueta*[Rose] K. Schum.),Ingwerextrakt (*Zingiber ssp*.,insbesondere *Zingiber officinale*) und Galangaextrakt (*Kaempferia galanga* oder *Alpinia galanga*).

Eine besonders geeignete Substanz ist das aus dem Ingwerextrakt stammende Gingerol, einsetzbar ist auch N-Ethyl-p-menthan-3-carboxamid (N-Ethyl-5-Methyl-2-isopropylcyclohexancarboxamid). Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N- vanillylamide, insbesondere Nonansäure-N- vanillylamid, 2-Alkensäureamide, insbesondere 2-Nonensäure-N-isobutylamid, 2-Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol.

Bei der Herstellung der Partikel kann die Einarbeitung von Wirkstoffen durch den Einsatz von Tensiden erleichtert werden. Diese Tenside verbleiben naturgemäß im Partikel. Zudem wird die spätere Beschichtung durch den Tensideinsatz stabilisiert, so daß bevorzugte erfindungsgemäße Partikel dadurch gekennzeichnet sind, daß der Partikelkern - bezogen auf sein Gewicht - zusätzlich 0,01 bis 5 Gew.-%, vorzugsweise 0,05 bis 2,5 Gew.-% und insbesondere 0,1 bis 1 Gew.-% Tensid(e) enthält.

Hierbei sind bevorzugte Tenside ausgewählt aus Fettalkoholsulfaten, insbesondere Natriumlaurylsulfat, und/oder Betainen, insbesondere Cocoamidopropylbetain.

Der Partikelkern ist erfindungsgemäß von einer Hülle umgeben, die - bezogen auf ihr Gewicht - 80 bis 100 Gew.-% wasserlösliche Kalium- und/oder Natriumsilikate und/oder SiO₂ enthält. 100 Gramm Hüllmaterial enthalten demnach
- 80 bis 100 Gramm wasserlösliches Kaliumsilikat oder
- 80 bis 100 Gramm wasserlösliches Natriumsilikat oder
- 80 bis 100 Gramm einer Mischung aus wasserlöslichem Kaliumsilikat und wasserlöslichem Natriumsilikat oder
- 80 bis 100 Gramm SiO₂ oder
- 80 bis 100 Gramm einer Mischung aus wasserlöslichem Kaliumsilikat und SiO₂ oder
- 80 bis 100 Gramm einer Mischung aus wasserlöslichem Natriumsilikat und SiO₂ oder
- 80 bis 100 Gramm einer Mischung aus wasserlöslichem Kaliumsilikat und wasserlöslichem Natriumsilikat und SiO₂
Wird SiO₂ als Hüllmaterial eingesetzt, sind erfindungsgemäß so genannte pyrogene Kieselsäuren bevorzugt. Besonders bevorzugt sind erfindungsgemäß Beschichtungen aus Natriumwassergläsern, die Anteile von SiO₂ enthalten können. Erfindungsgemäß bevorzugte Partikel sind dadurch gekennzeichnet, daß die Hülle wasserlösliche Kalium- und/oder Natriumsilikate ("Wassergläser") enthält, die ein Modul SiO₂ zu MeO₂ von M > 1, bevorzugt von M > 2 und besonders bevorzugt M > 3 aufweisen.

Kern und Hülle bilden gemeinsam den erfindungsgemäßen beschichteten Partikel. Bei den beschichteten Partikeln können die Massenverhältnisse zwischen Kern und Hülle (und damit die Dicke der Hülle) variieren. Es ist jedoch bevorzugt, daß der Kern mindestens 50 Gew.-%, vorzugsweise mindestens 60 Gew.-% und insbesondere mindestens 70 Gew.-% des Gesamtgewichts des beschichteten Partikels ausmacht. Noch weiter bevorzugte erfindungsgemäße Partikel sind dadurch gekennzeichnet, daß die Hülle 0,1 bis 25 Gew. %, vorzugsweise 0,5 bis 10 Gew. % und besonders bevorzugt 1 bis 5 Gew. % des beschichteten Partikels ausmacht.

Die erfindungsgemäßen Partikel können weitere Inhaltsstoffe in geringen Mengen enthalten. Je nach Art und Menge des Inhaltsstoffes können diese in den Kern oder in die Hülle oder in beide Regionen der beschichteten Partikel eingearbeitet werden.
Eine besonders bevorzugte Gruppe von Inhaltsstoffen, die vorzugsweise in die Hülle der erfindungsgemäßen Partikel eingearbeitet werden kann, sind Farbstoffe. Hier sind erfindungsgemäße Partikel bevorzugt, die mindestens einen Farbstoff aus der Gruppe der kationischen (basischen) Farbstoffe, vorzugsweise Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74, C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf den Partikel inklusive Beschichtung, enthalten. Wie bereits erwähnt, sind die Farbstoffe vorzugsweise in der Beschichtung enthalten.

Die erfindungsgemäßen Partikel zeigen eine geringe Ausblutungsneigung auch in flüssigen Medien und eignen sich hervorragend zur Inkorporation in Kosmetika. Ein weiterer Gegenstand der vorliegenden Erfindung ist daher ein kosmetisches Haar- und/oder Körperreinigungsmittel, enthaltend in einem kosmetisch akzeptablen Träger - bezogen auf sein Gewicht -
a) 0,5 bis 20 Gew.-% erfindungsgemäße Partikel und
b) 5 bis 50 Gew.-% Tensid(e).
Die erfindungsgemäßen Partikel wurden weiter oben detailliert beschrieben. Es folgt eine Beschreibung der Tenside als zweitem zwingenden Inhaltstoff sowie weiterer optionaler Inhaltsstoffe. Die erfindungsgemäßen Mittel enthalten 0,5 bis 20 Gew.-% mindestens eines Tensids. Besonders bevorzugt ist der Einsatz von amphoteren und/oder zwitterionischen Tensiden.
Besonders bevorzugte erfindungsgemäße Mittel sind dadurch gekennzeichnet, daß sie amphotere(s) Tensid(e) aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe,Alkylaminoessigsäuren mit jeweils etwa 8 bis 24 C-Atomen in der Alkylgruppe, N-Kokosalkylaminopropionat, Kokosacylamino-ethylaminopropionat C₁₂ - C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispiels-weise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder AcylgruppeKokosacylaminoethylhydroxy-ethylcarboxymethylglycinatder unter der INCI-Bezeichnung Cocamidopropyl Betain bekannten Verbindungen, der unter der INCI-Bezeichnung Disodium Cocoamphodiacetate bekannten Verbindungenenthalten, wobei bevorzugte Mittel das bzw. die amphotere(n) Tensid(e) in Mengen von 0,5 bis 9 Gew.-%, vorzugsweise von 0,75 bis 8 Gew.-% und insbesondere von 1 bis 7,5 Gew.-%, jeweils bezogen auf das gesamte Mittel, enthalten.

Bevorzugte erfindungsgemäße Mittel enthalten als amphotere Tenside Betaine der Formel (I) 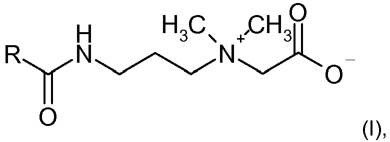 in der R für einen geradkettigen oder verzweigten, gesättigten oder ein- bzw. mehrfach ungesättigten Alkyl- oder Alkenlyrest mit 8 bis 24 Kohlenstoffatomen steht. Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-NH-(CH₂)₃N⁺(CH₃)₂CH₂COO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻
Besonders bevorzugt werden Tenside der Formel (I) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (I) enthalten. Zusätzlich zu dem bzw. den Amphotensiden der Formel (I) oder an deren Stelle können die erfindungsgemäßen Mittel mit besonderem Vorzug als amphotere Tenside Betaine der Formel (II) 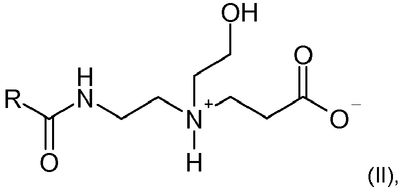 enthalten, in der R für einen geradkettigen oder verzweigten, gesättigten oder ein- bzw. mehrfach ungesättigten Alkyl- oder Alkenlyrest mit 8 bis 24 Kohlenstoffatomen steht. Diese Tenside werden nach der INCI-Nomenklatur als Amphoacetate bezeichnet, wobei die Vertreter, die sich von Kokosfettsäuren ableiten bevorzugt sind und als Cocoamphoactetate bezeichnet werden. Aus herstellungstechnischen Gründen enthalten Tenside dieses Typs immer auch Betaine der Formel (IIa) 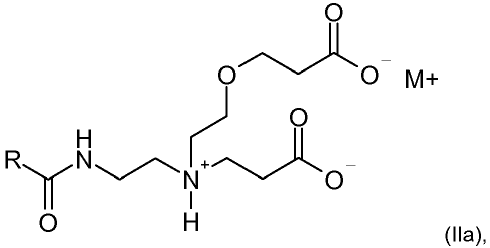 in der R für einen geradkettigen oder verzweigten, gesättigten oder ein- bzw. mehrfach ungesättigten Alkyl- oder Alkenlyrest mit 8 bis 24 Kohlenstoffatomen und M für ein Kation steht. Diese Tenside werden nach der INCI-Nomenklatur als Amphodiacetate bezeichnet, wobei die Vertreter, die sich von Kokosfettsäuren ableiten, bevorzugt sind und als Cocoamphodiactetate bezeichnet werden.

Besonders bevorzugt werden erfindungsgemäß Tenside der Formel (II) eingesetzt, die ein Gemisch der folgenden Vertreter sind:
H₃C-(CH₂)₇-C(O)-NH-(CH₂)₂NH⁺(CH₂CH₂OH)CH₂CH₂COO⁻
H₃C-(CH₂)₉-C(O)-NH-(CH₂)₂NH⁺(CH₂CH₂OH)CH₂CH₂COO⁻
H₃C-(CH₂)₁₁-C(O)-NH-(CH₂)₂NH⁺(CH₂CH₂OH)CH₂CH₂COO⁻
H₃C-(CH₂)₁₃-C(O)-NH-(CH₂)₂NH⁺(CH₂CH₂OH)CH₂CH₂COO⁻
H₃C-(CH₂)₁₅-C(O)-NH-(CH₂)₂NH-(CH₂CH₂OH)CH₂CH₂COO⁻
H₃C-(CH₂)₇-CH=CH-(CH₂)₇-C(O)-NH-(CH₂)₂NH⁺(CH₂CH₂OH)CH₂CH₂COO⁻
Besonders bevorzugt werden Tenside der Formel (II) innerhalb engerer Mengenbereiche eingesetzt. Hier sind erfindungsgemäße Mittel bevorzugt, die - bezogen auf ihr Gewicht - 0,25 bis 8 Gew.-%, weiter bevorzugt 0,5 bis 7 Gew.-%, weiter bevorzugt 0,75 bis 6,5 Gew.-% und insbesondere 1 bis 5,5 Gew.-% Tensid(e) der Formel (II) enthalten.

Zusammenfassend sind erfindungsgemäße Mittel bevorzugt, bei denen der Rest R in den Formeln (I) und (II) ausgewählt ist aus H₃C-(CH₂)₇-, H₃C-(CH₂)₉-, H₃C-(CH₂)₁₁-, H₃C-(CH₂)₁₃-, H₃C-(CH₂)₁₅-, H₃C-(CH₂)₇-CH=CH-(CH₂)₇-, oder Mischungen aus diesen.

Zusätzlich zu den genannten Tensiden können die erfindungsgemäßen Mittel auch anionische, kationische und gegebenenfalls auch weitere amphotere bzw. zwitterionische bzw. nichtionische Tenside enthalten.
Es ist erfindungsgemäß bevorzugt, auf den Einsatz von Alkyl- und/oder Alkenylsulfaten zu verzichten, um ausgesprochen milde Eigenschaften der Mittel zu erhalten. Bevorzugte erfindungsgemäße Mittel sind dadurch gekennzeichnet, daß sie keine Alkyl- und Alkenylsulfate enthalten. Im Hinblick auf besonders milde Formulierungen ist auch der Einsatz von Alkyl- oder Alkenylethersulfaten erfindungsgemäß nicht bevorzugt. Hier sind Mittel bevorzugt, die keine Alkyl- und Alkenylethersulfate enthalten. Generell ist der Einsatz von Sulfattensiden in den erfindungsgemäßen Mitteln eher kontraindiziert, so daß besonders bevorzugte erfindungsgemäße Mittel sulfatfrei sind.

Besonders bevorzugte nichtionische Tenside sind Alkylpolyglycoside. Demnach sind erfindungsgemäße Mittel bevorzugt, die als nichtionische Tenside Alkylpolyglycoside der allgemeinen Formel RO-(Z)ₓ enthalten, wobei R für Alkyl, Z für Zucker sowie x für die Anzahl der Zuckereinheiten steht.
Die Pflegeffekte der erfindungsgemäßen Mittel lassen sich noch weiter verstärken, indem Pflege-Enhancer eingesetzt werden. Vorzugsweise werden diese aus bestimmten Gruppen an sich bekannter Pflegestoffe ausgewählt, da diese Pflege-Enhancer formulierungstechnisch und vom Pflegeffekt hervorragend mit den erfindungsgemäß eingesetzten Pflanzenextrakten harmonieren.
Erfindungsgemäß bevorzugte Mittel sind dadurch gekennzeichnet, daß sie zusätzlich mindestens einen Pflege-Enhancer aus der Gruppe
i. L-Carnitin und/oder seiner Salze;
ii. Panthenol und/oder Panthothensäure;
iii. der 2-Furanone und/oder deren Derivate, insbesondere Pantolacton;
iv. Taurin und/oder seiner Salze;
v. Niacinamid;
vi. Ubichinon
vii. Ectoin;
viii. Allantoin;
enthalten.

In erfindungsgemäßen Haarbehandlungsmitteln dieser Ausführungsform wird die Tensid-/Pflanzenextraktkombination mit mindestens einem Pflege-Enhancer kombiniert, der ausgewählt ist aus L-Carnitin und/oder seinen Salzen, Panthenol und/oder Panthothensäure, 2-Furanonen und/oder deren Derivaten, insbesondere Pantolacton, Taurin und/oder seinen Salzen, Niacinamid, Ubichinonen, Ectoin, Allantoin, Extrakten von Echinacea. Diese Pflege-Enhancer sind im Prioritätsdokument detailliert beschrieben.
Erfindungsgemäße bevorzugte Mittel sind dadurch gekennzeichnet, daß sie - bezogen auf ihr Gewicht - 0,001 bis 10 Gew.-%, vorzugsweise 0,005 bis 7,5 Gew.-%, besonders bevorzugt 0,01 bis 5 Gew.-% und insbesondere 0,05 bis 2,5 Gew.-% L-Carnitin oder L-Carnitinderivate enthalten, wobei bevorzugte L-Carnitinderivate ausgewählt sind aus Acetyl-L-Carnitin, L-Carnitin-Fumarat, L-Carnitin-Citrat, Lauroyl- L-Carnitin und insbesondere L-Carnitin-Tartrat.
Erfindungsgemäße bevorzugte Mittel sind dadurch gekennzeichnet, daß sie - bezogen auf sein Gewicht -0,01 bis 5 Gew.-%, vorzugsweise 0,05 bis 2,5 Gew.-%, besonders bevorzugt 0,1 bis 1,5 Gew.-% und insbesondere 0,25 bis 1 Gew.-% Panthenol ((±)-2,4-Dihydroxy-*N*-(3-hydroxypropyl)-3,3-dimethyl-butyramid) enthalten.
Erfindungsgemäß bevorzugte Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% mindestens eines 2-Furanonderivats der Formel (Fur-I) und/oder der Formel (Fur-II) 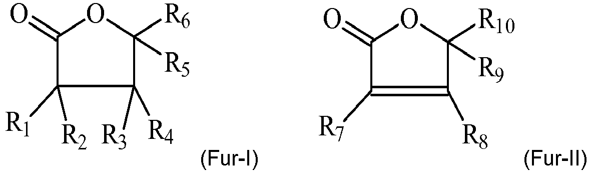 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht.
Ein weiterer, bevorzugter einsetzbarer Pflege-Enhancer, der aktivierende Eigenschaften besitzt, ist das Taurin. Erfindungsgemäß bevorzugte Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).
Zusammenfassend sind erfindungsgemäße Mittel bevorzugt, die - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel -2,4-Dihydroxy-*N*-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.
Es hat sich gezeigt, daß bestimmte Chinone eine besondere Eignung als Pflege-Enhancer besitzen. Als weiteren Pflege-Enhancer können die erfindungsgemäßen Mittel daher 0,0001 bis 5 Gew.-% mindestens eines Biochinons der Formel (Ubi) 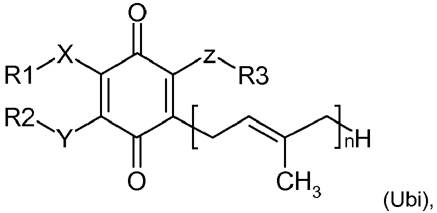 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂, -CH(CH₃)₂,-(CH₂)₃CH₃, -CH(CH₃)CH₂CH₃, -CH₂CH(CH₃)₂, -C(CH₃)₃
- R⁴: steht für -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.
Besonders bevorzugte erfindungsgemäße Mittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 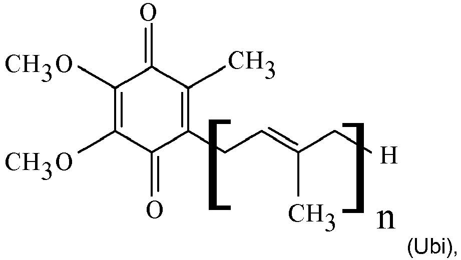 in der n für die Werte = 6, 7, 8, 9 oder 10, besonders bevorzugt für 10 (Coenzym Q10) steht.
Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, daß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ubi-b) enthalten 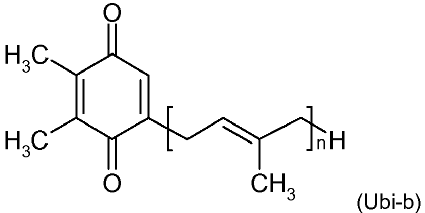 in der n für Werte von 1 bis 20, vorzugsweise von 2 bis 15 und insbesondere für 5, 6, 7, 8, 9, 10 steht, wobei besonders bevorzugt Mittel Plastochinon PQ-9 der Formel 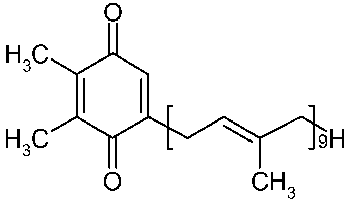 enthalten.
Als weiteren Pflege-Enhacer können die erfindungsgemäßen Mittel Ectoin enthalten. Erfindungsgemäß bevorzugte Mittel sind dadurch gekennzeichnet, daß sie - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% (S)-2-Methyl-1,4,5,6-tetrahydro-4-pyrimidincarbonsäure (Ectoin) sowie die physiologisch verträglichen Salze dieser Verbindung und/oder (S,S)-5-Hydroxy-2-methyl-1,4,5,6-tetrahydro-4-pyrimidincarbonsäure (Hydroxyectoin) sowie die physiologisch verträglichen Salze dieser Verbindung, enthalten.
Erfindungsgemäße besonders bevorzugte Mittel enthalten - bezogen auf ihr Gewicht - 0,001 bis 10 Gew.-%, vorzugsweise 0,01 bis 5 Gew.-%, besonders bevorzugt 0,05 bis 2,5 Gew.-% und insbesondere 0,1 bis 1 Gew.-% 5-Ureidohydantoin (Allantoin).
Zur Verbesserung der Elastizität und Festigung der inneren Struktur der mit erfindungsgemäßen Mitteln behandelter Haare können die erfindungsgemäßen Mittel Purin und/oder Purinderivate als Pflege-Enhancer enthalten. Insbesondere die Kombination von Purin und/oder Purinderivaten mit Ubichinonen und/oder Plastochinonen als Pflege-Enhancer führt dazu, daß die mit entsprechenden Mitteln behandelten Haare unter anderem höhere Meßwerte bei der Differenzthermoanalyse und verbesserte Naß- und Trockenkämmbarkeiten zeigen.
Bevorzugte erfindungsgemäße Mittel enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.
Unter Purin, den Purinen und den Purinderivaten sind erfindungsgemäß einige Vertreter besonders bevorzugt. Erfindungsgemäß bevorzugte Mittel sind dadurch gekennzeichnet, daß sie als Pflege-Enhancer - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 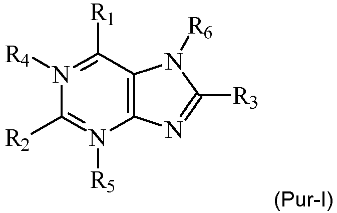 in der die Reste R¹, R² und R³ unabhängig voneinander ausgewählt sind aus -H, -OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).
Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis von Purin(derivat(en)) und Biochinon(en) 10:1 bis 1:100, vorzugsweise 5:1 bis 1:50, besonders bevorzugt 2:1 bis 1:20 und insbesondere 1:1 bis 1:10 beträgt.
Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten. Erfindungsgemäß besonders bevorzugte Mittel sind dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Flavonoide, insbesondere Flavonole, besonders bevorzugt 3,3',4',5,7-Pentahydroxyflavon (Quercetin) und/oder 3,3',4',5,7-Pentahydroxyflavon-3-*O-*rutinosid (Rutin), enthalten.
Bevorzugt ist auch der Einsatz von Bisabolol und/oder Bisabololoxiden als Pflege-Enhancer in den erfindungsgemäßen Mitteln. Hier sind erfindungsgemäße Mittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol 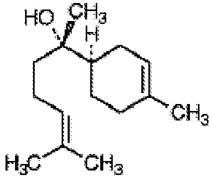 enthalten.
Auch Creatin eignet sich erfindungsgemäß als Pflege-Enhancer. Besonders bevorzugte erfindungsgemäße Mittel enthalten - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% *N*-Methyl-guanidino-essigsäure (Creatin).
Die erfindungsgemäßen Mittel können zusätzlich zu den vorstehend genannten Inhaltsstoffen und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft. Diese Stoffe werden nachstehend beschrieben: Propecia (Finasterid) ist das zur Zeit einzige Präparat, das weltweit zugelassen ist und für das in zahlreichen Studien eine Wirksamkeit und Verträglichkeit nachgewiesen wurde. Propecia bewirkt, daß sich weniger DHT aus Testosteron bilden kann. Minoxidil ist mit oder ohne ergänzende Zusatzstoffe das wohl älteste nachweislich wirkende Haarwuchsmittel. Zur Behandlung von Haarausfall darf es nur zur äußeren Anwendung verwendet werden. Es gibt Haarwasser, die 2%-5% Minoxidil enthalten, außerdem Gels mit bis zu 15% Minoxidil. Die Wirksamkeit nimmt mit der Dosierung zu, in Haarwassern ist Minoxidil jedoch nur bis zu 5% Anteil löslich. In vielen Ländern sind Haarwasser mit bis zu 2% Minoxidilgehalt verschreibungsfrei erhältlich. Zur Bekämpfung der hormonellen Einflüße auf die Haarfollikel kann zur äußeren Anwendung Spironolactone in Form von Haarwasser und in Kombination mit Minoxidil angewandt werden. Spironolactone wirkt als Androgen-Rezeptor-Blocker, dh. die Bindung von DHT an die Haarfollikel wird verhindert.
Zusammenfassend sind erfindungsgemäße Haarbehandlungsmittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten. Durch zusätzliche Antischuppenwirkstoffe (beispielsweise Climbazol, Piroctone Olamine oder Zink-Pyrithion) wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, daß die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.
Zusammenfassend sind erfindungsgemäße Mittel bevorzugt, die zusätzlich - bezogen auf ihr Gewicht - 0,001 bis 5 Gew.-% Antischuppenwirkstoffe, insbesondere Piroctone Olamine (1-Hydroxy-4-methyl-6-(2,4,4-trirnethylpentyl)pyridin-2(1H)-on, Verbindung mit 2-Aminoethanol, 1:1) und/oder Zink-Pyrithion und/oder Selensulfid und/oder Climbazol und/oder Salicylsäure oder Fumarsäure enthalten.

Zusätzlich zu den Pflege-Enhancern können di erfindungsgemäßen Mittel weitere Pflegestoffe enthalten. Deren Anwesenheit ist für die Erzielung der erfindungsgemäßen Effekte nicht zwingend erforderlich, doch können weitergehende Effekte, wie ein angenehmer Griff oder eine angenehme Applikationshaptik aus dem Einsatz dieser Pflegestoffe resultieren. Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat und 0,1 bis 0,25 Gew.-% Glycin,
- 0,005 bis 0,015 Gew.-% Coffein und 0,75 bis 1,5 Gew.-% Saccharose und 0,1 bis 0,25 Gew.-% Glycin,
- 0,005 bis 0,015 Gew.-% Coffein und 0,75 bis 1,5 Gew.-% Fructose und 0,1 bis 0,25 Gew.-% Glycin,
- 0,005 bis 0,015 Gew.-% Coffein und 0,75 bis 1,5 Gew.-% Glucosemonohydrat und 0,1 bis 0,25 Gew.-% Alanin,
- 0,005 bis 0,015 Gew.-% Coffein und 0,75 bis 1,5 Gew.-% Saccharose und 0,1 bis 0,25 Gew.-% Alanin,
- 0,005 bis 0,015 Gew.-% Coffein und 0,75 bis 1,5 Gew.-% Fructose und 0,1 bis 0,25 Gew.-% Alanin,
- 0,005 bis 0,015 Gew.-% Coffein und 0,75 bis 1,5 Gew.-% Glucosemonohydrat und 0,1 bis 0,25 Gew.-% Valin,
- 0,005 bis 0,015 Gew.-% Coffein und 0,75 bis 1,5 Gew.-% Saccharose und 0,1 bis 0,25 Gew.-% Valin,
- 0,005 bis 0,015 Gew.-% Coffein und 0,75 bis 1,5 Gew.-% Fructose und 0,1 bis 0,25 Gew.-% Valin.
Eine besonders bevorzugte Gruppe von Inhaltsstoffen stellen die Silikone dar. Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.
Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov®68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls® PGPH),
- Lineare und verzweigte Fettsäuren mit 8 bis 30 C - Atomen und deren Na-, K-, Ammonium-, Ca-, Mg- und Zn - Salze.
Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel. Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn zusätzlich zu dem bzw. den Polymer(en) aus der Gruppe der kationischen und/oder amphoteren Polymere weitere Polymere (G) in den erfindungsgemäßen Mitteln enthalten sind. In einer bevorzugten Ausführungsform werden den erfindungsgemäßen Mitteln daher weitere Polymere zugesetzt, wobei sich sowohl anionische als auch nichtionische Polymere als wirksam erwiesen haben.

Als ganz besonders wirkungsvoll haben sich anionische Polymere erwiesen, die als alleiniges oder CoMonomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt ist das Homopolymer der 2-Acrylamido-2-methylpropansulfonsäure, das beispielsweise unter der Bezeichnung Rheothik®11-80 im Handel erhältlich ist.
Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.
Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel®305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.
Auch die unter der Bezeichnung Simulgel®600 als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol® im Handel erhältlich. Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze® QM im Handel erhältlich.
Die erfindungsgemäßen Mittel können in einer weiteren Ausführungsform nichtionogene Polymere (G4) enthalten.
Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten. Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Die erfindungsgemäßen Partikel lassen ich nicht nur in Haar- und/oder Körperreinigungsmittel inkorporieren, sondern auch in andere Kosmetika, beispielsweise Pflegemittel für Haut oder Haare wie Cremes, Lotionen oder Conditioner und Haarkuren.

Ein besonders bevorzugtes Anwendungsgebiet für die erfindungsgemäßen Partikel sind kosmetische Mittel für die Mund- und Zahnpflege. Hier eröffnen die erfindungsgemäßen Partikel wegen ihrer Stabilität und physiologischen Verträglichkeit ganz besondere optische Differenzierungen.

Ein weiterer Gegenstand der vorliegenden Erfindung sind daher Mund- und Zahnpflege- und -reinigungsmittel, enthaltend - bezogen auf ihr Gewicht -
a) 0,5 bis 20 Gew.-% erfindungsgemäße Partikel und
b) 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die erfindungsgemäßen Mund- und Zahnpflege- und -reinigungsmittel sind vorzugsweise fließfähig. Unter "fließfähig" werden im Rahmen der vorliegenden Erfindung Zusammensetzungen verstanden, die bei 20°C und 1013,25 mbar Viskositäten zwischen 0,001 und 1.000.000 mPas (=1 µPas bis 1 kPas) aufweisen. Bevorzugte erfindungsgemäße Zubereitungen weisen Viskositäten zwischen 0,1 und 500.000 mPas, vorzugsweise zwischen 1 und 250.000 mPas und insbesondere zwischen 100 und 75.000 mPas auf. In einer weiter bevorzugten Ausführungsform der Erfindung weisen die Mittel eine Viskosität [mPas] im Bereich von 8.000 bis 45.000 mPas auf (gemessen mit Brookfield RVF; Spindel 4/4UpM bei 20°C).

Die erfindungsgemäßen Zusammensetzungen sind vorzugsweise wäßrige Mittel, deren wäßriger Träger vorzugsweise mindestens 5 Gew.-% Wasser enthält. Dessen Menge kann ja nach weiteren Inhaltsstoffen variieren, besonders bevorzugte erfindungsgemäße Mund und Zahnpflege- und -reinigungsmittel sind dadurch gekennzeichnet, daß sie weniger als 60 Gew.-%, vorzugsweise weniger als 55 Gew.-%, besonders bevorzugt weniger als 50 Gew.-% und insbesondere weniger als 45 Gew.-% Wasser enthalten.

Als zweiten wesentlichen Inhaltsstoff enthalten die erfindungsgemäßen Mund- und Zahnpflege- und - reinigungsmittel 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol.

Sorbit (auch als Glucit bezeichnet) ist ein Zuckeralkohol von Glucose, also ein Hexit. Sorbit ist durch Hydrierung von Glucose herstellbar, spaltet intramolekular relativ leicht ein oder zwei Moleküle Wasser ab und bildet cyclische Ether. Glycerin (1,2,3-Propantriol, 1,2,3-Trihydroxypropan, Glycerol, Ölsüß, INCI-Bezeichnung: Glycerin, E 422) ist eine farblose, klare, schwerbewegliche, geruchlose, süß schmeckende, hygroskopische Flüssigkeit, die mit Wasser und Alkohol in jedem Verhältnis mischbar ist. 1,2-Propylenglykol (1,2-Propandiol) ist eine farblose und stark hygroskopische Flüssigkeit, die in jedem Verhältnis mit Wasser und Alkoholen (wie Methanol, Ethanol, Propanolen, Butanolen) mischbar ist. Technisches 1,2-Propandiol ist ein Racemat aus (-)-(*R*)- und (+)-(*S*)-1,2-Prpylenglycol.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.
Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen.
Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden. Besonders bevorzugt ist der Einsatz von Sorbit, so daß Mittel, die außer Sorbit keine anderen mehrwertigen Alkohole enthalten, besonders bevorzugt sind.

Der Einsatz von Putzkörpern (Abrasiva) ist ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel). Als Poliermittel eignen sich prinzipiell alle für Zahnpasten bekannten Reibkörper, insbesondere solche, die keine Calciumionen enthalten. Bevorzugt geeignete Poliermittelkomponenten sind daher Kieselsäuren, Aluminiumhydroxid, Aluminiumoxid, Natrium-aluminiumsilikate, organische Polymere oder Gemische solcher Reibkörper. Calciumhaltige Polierkomponenten wie z.B. Kreide, Calciumpyrophosphat, Dicalcium-phosphat-dihydrat können aber in Mengen bis zu 5 Gew.-% - bezogen auf die Gesamtzusammensetzung - enthalten sein. Der Gesamtgehalt an Poliermitteln liegt vorzugsweise im Bereich von 5 - 50 Gew.-% des Zahnpflegemittels. Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich. Andere Fällungskieselsäuren dieser Art sind Sident® 8 (DEGUSSA) und Sorbosil® AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen. Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungs-kieselsäuren mit einer BET-Oberfläche von 150 --250 m²/g zu, z.B. die Handelsprodukte Sipernat 22 LS oder Sipernat® 320 DS. Als weitere Poliermittelkomponente kann auch z.B. Aluminiumoxid in Form von schwach calcinierter Tonerde mit einem Gehalt an - und - Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich. Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄·2H₂O), Natriumaluminiumsilikate, insbesondere Zeolith A, organische Polymere, insbesondere Polymethacrylate oder Gemische dieser Reibkörper,vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel, insbesondere Zahnpasten, können z.B. auch Substanzen enthalten, die gegen Plaque und/oder Zahnstein wirksam sind.

In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Kaliumnitrat (E 252), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden. Bevorzugte Stoffe sind ausgewählt aus p-Hydroxybenzoesäuremethyl-, -ethyl- oder propylester, Natriumsorbat, Natriumbenzoat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Erfindungsgemäß bevorzugte Mittel enthalten zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel. Die angegebenen Mengen beziehen sich auf die Gesamtmenge an Antiplaque-Wirkstoffen, wobei einzelne Wirkstoffe vorzugsweise in engeren Mengenbereichen eingesetzt werden. Erfindungsgemäß bevorzugte Mittel enthalten beispielsweise 0,1 bis 2 Gew.-%, vorzugsweise 0,2 bis 1,75 Gew.-%, weiter bevorzugt 0,3 bis 1,5 Gew.-% und insbesondere 0,4 bis 1,0 Gew.-% Natriumbenzoat. Weiter bevorzugte erfindungsgemäße Mittel sind dadurch gekennzeichnet, daß sie 0,005 bis 0,1 Gew.-%, vorzugsweise 0,01 bis 0,075 Gew.-% und insbesondere 0,02 bis 0,05 Gew.-% Chlorhexidin enthalten. Chlorhexidin wird vorzugsweise gemeinsam mit Alkylpolyglycosiden (APG) eingesetzt, wobei in bevorzugten erfindungsgemäßen Mitteln als Alkylglykoside solche mit 8-18 C-Atomen in der Alkylgruppe und einem mittleren Oligomerisationsgrad des Glycosidrestes von 1-3 in einer Menge von 0,025 bis 2,5 Gew.-% enthalten sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können. Erfindungsgemäß bevorzugte Mittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein. Erfindungsgemäße Mittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000. Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten. Besonders bevorzugte erfindungsgemäße Mund und Zahnpflege- und -reinigungsmittel enthalten 0,5 bis 9,5 Gew.-%, vorzugsweise 1 bis 9 Gew.-%, weiter bevorzugt 2 bis 8 Gew.-%, noch weiter bevorzugt 3 bis 7 Gew.-% und insbesondere 4 bis 6 Gew.-% Kaliumsalz(e).

Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten. Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol D - (+) - 2,4-Dihydroxy-N-(3-hydroxypropyl)-3,3-dimethyl-butyramid zeigt eine der Pantothensäure entsprechende biologische Aktivität. Die erfindungsgemäßen Zahnpflegemittel enthalten bevorzugt 0,05 - 5 Gew.-% Panthenol oder ein Salz der Pantothensäure. Retinol (3,7-Dimethyl-9-(2,6,6-trimethyl-1-cyclohexenyl)-2,4,6,8-nonatetraen-1-ol ist der internationale Freiname für Vitamin A1. Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. Erfindungsgemäß bevorzugte Mund- und Zahnpflege- und -reinigungsmittel enthalten 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat.

Die erfindungsgemäßen Zahnpflegemittel können z.B. durch Zusatz von Aromaölen und Süßungsmitteln in ihren organoleptischen Eigenschaften verbessert werden. Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten. Als wasserunlösliche Aromaöle kommen alle für Mund- und Zahnpflegemittel gebräuchlichen natürlichen und synthetischen Aromen infrage. Natürliche Aromen können sowohl in Form der aus den Drogen isolierten ätherischen Öle als auch in Form der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton. Besonders bevorzugte erfindungsgemäße Mund- und Zahnpflege- und -reinigungsmittel sind dadurch gekennzeichnet, daß sie - bezogen auf ihr Gewicht - 0,05 bis 10 Gew.-%, vorzugsweise 0,1 bis 7,5 Gew.-%, besonders bevorzugt 0,15 bis 5 Gew.-%, weiter bevorzugt 0,2 bis 4 Gew.-% und insbesondere 0,25 bis 3 Gew.-% mindestens eines Aromas aus der Gruppe Pfefferminz, Spearmint, Menthol, Limone, Limette, Apfel, Orange, Zitrone, Erdbeere, Aloe Vera, Kaugummi enthalten.

Wie bereits weiter oben erwähnt, sind diese Aromen vorzugsweise Bestandteil der beschichteten Partikel, die erfindungsgemäß in den Mund- und Zahnpflege- und -reinigungsmitteln enthalten sind.

Geeignete Süßungsmittel sind z.B. Saccharin-Natrium, Natrium-Cyclamat, Sucrose, Lactose, Meltose, Fructose. Weitere übliche Hilfs- und Zusatzstoffe für Zahnpasten sind oberflächenaktive Stoffe, bevorzugt anionische, zwitterionische, amphotere, nicht- ionische Tenside oder eine Kombination mehrerer verschiedener Tenside, Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol, Pigmente, wie z.B. Titandioxid, Farbstoffe, Puffersubstanzen, z.B. primäre, sekundäre oder tertiäre Alkaliphosphate oder Citronen- säure-/Na-Citrat, weitere wundheilende oder entzündungshemmende Stoffe, z.B. Allantoin, Harnstoff, Azulen, Kamillewirkstoffe, Acetylsalicylsäurederivate oder Rhodanid, weitere Vitamine wie z.B. Ascorbinsäure, Biotin, Tocopherol oder Rutin, Mineralsalze wie z.B. Mangan-, Zink- oder Magnesiumsalze.

Eine weitere wichtige Gruppe von Inhaltstoffen, die in den erfindungsgemäßen Mitteln enthalten sein kann, sind die sogenannten bioaktiven Gläser. Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, daß es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet.
Erfindungsgemäß besonders bevorzugte Mittel sind dadurch gekennzeichnet, daß sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.

Besonders bevorzugte erfindungsgemäße Mund- und Zahnpflege- und -reinigungsmittel sind dadurch gekennzeichnet, daß das bioaktive Glas - bezogen auf sein Gewicht - folgende Zusammensetzung aufweist:

| | | | |
|---|---|---|---|
| SiO₂ | 35 bis 60 Gew.-%, | vorzugsweise | 40 bis 60 Gew.-%, |
| Na₂O | 0 bis 35 Gew.-%, | vorzugsweise | 5 bis 30 Gew.-%, |
| K₂O | 0 bis 35 Gew.-%, | vorzugsweise | 0 bis 20 Gew.-%, |
| P₂O₅ | 0 bis 10 Gew.-%, | vorzugsweise | 2 bis 10 Gew.-%, |
| MgO | 0 bis 10 Gew.-%, | vorzugsweise | 0 bis 5 Gew.-%, |
| CaO | 0 bis 35 Gew.-%, | vorzugsweise | 5 bis 30 Gew.-%, |
| Al₂O₃ | 0 bis 25 Gew.-%, | vorzugsweise | 0 bis 5 Gew.-%, |
| B₂O₃ | 0 bis 25 Gew.-%, | vorzugsweise | 0 bis 5 Gew.-%, |
| TiO₂ | 0 bis 10 Gew.-%, | vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und -reinigungsmittel dadurch gekennzeichnet, daß das antimikrobielle Glas Teilchengrößen < 10 µm, vorzugsweise von 0,5 bis 4 µm, besonders bevorzugt von 1 bis 2 µm, aufweist.

## Patentansprüche

1. Beschichtete Duft- und/oder Aromapartikel, umfassend einen Partikelkern und eine diesen Kern umgebende Hülle, **dadurch gekennzeichnet, dass**
a) der Partikelkern - bezogen auf sein Gewicht - 80 bis 99,99 Gew.-% Silikat(e) enthält und
b) der Partikelkern - bezogen auf sein Gewicht - 0,01 bis 20 Gew.-% Duftstoff(e) und/oder Aromen enthält und
c) die den Kern umgebende Hülle - bezogen auf ihr Gewicht - 80 bis 100 Gew.-% wasserlösliche Kalium- und/oder Natriumsilikate enthält, wobei
das bzw. die Silikat(e) des Partikelkerns Partikelgrößen zwischen 10 und 1000 µm aufweisen.

2. Partikel nach Anspruch 1, **dadurch gekennzeichnet, dass** das bzw. die Silikat(e) eine gewichtsmittlere Partikelgrößenverteilung aufweisen, bei der > 70 Gew.-%, vorzugsweise > 80 Gew.-% und insbesondere > 90 Gew.-% der Teilchen Größen zwischen 100 und 700 µm aufweisen.

3. Partikel nach einem der Ansprüche 1 oder 2, **dadurch gekennzeichnet, dass** der Partikelkern zusätzlich Titandioxid enthält.

4. Partikel nach einem der Ansprüche 1 bis 3, **dadurch gekennzeichnet, dass** der Partikelkern - bezogen auf sein Gewicht - 0,05 bis 19 Gew.-%, vorzugsweise 0,1 bis 18 Gew.-%, besonders bevorzugt 0,5 bis 17 Gew.-%, weiter bevorzugt 0,75 bis 16 Gew.-% und insbesondere 1 bis 15 Gew.-% mindestens eines Aromas aus der Gruppe Pfefferminz, Spearmint, Menthol, Limone, Limette, Apfel, Orange, Zitrone, Erdbeere, Aloe Vera enthält.

5. Partikel nach einem der Ansprüche 1 bis 4, **dadurch gekennzeichnet, dass** der Partikelkern - bezogen auf sein Gewicht - zusätzlich 0,01 bis 5 Gew.-%, vorzugsweise 0,05 bis 2,5 Gew.-% und insbesondere 0,1 bis 1 Gew.-% Tensid(e) enthält.

6. Partikel nach Anspruch 5, **dadurch gekennzeichnet, dass** das als Tensid(e) Fettalkoholsulfate, insbesondere Natriumlaurylsulfat, und/oder Betaine, insbesondere Cocoamidopropylbetain, eingesetzt werden.

7. Partikel nach einem der Ansprüche 1 bis 6, **dadurch gekennzeichnet, dass** die Hülle wasserlösliche Kalium- und/oder Natriumsilikate ("Wassergläser") enthält, die ein Modul Si0₂ zu Me0 von M > 1, bevorzugt von M > 2 und besonders bevorzugt M > 3 aufweisen.

8. Partikel nach einem der Ansprüche 1 bis 7, **dadurch gekennzeichnet, dass** die Hülle 0, 1 bis 25 Gew. %, vorzugsweise 0,5 bis 10 Gew. % und besonders bevorzugt 1 bis 5 Gew. % des beschichteten Partikels ausmacht.

9. Partikel nach einem der Ansprüche 1 bis 8, **dadurch gekennzeichnet, dass** er mindestens einen Farbstoff aus der Gruppe der kationischen (basischen) Farbstoffe, vorzugsweise Basic Blue 6, C.I.-Nο. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74, C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino-2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2'-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf den Partikel inklusive Beschichtung, enthält.

10. Kosmetisches Haar- und/oder Körperreinigungsmittel, enthaltend in einem kosmetisch akzeptablen Träger - bezogen auf sein Gewicht -
a) 0,5 bis 20 Gew.-% Partikel nach einem der Ansprüche 1 bis 9 und
b) 5 bis 50 Gew.-% Tensid(e).

11. Mund- und Zahnpflege- und -reinigungsmittel, enthaltend - bezogen auf sein Gewicht -
a) 0,5 bis 20 Gew.-% Partikel nach einem der Ansprüche 1 bis 9 und
b) 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit, Glycerin, 1,2-Propylenglycol.

12. Mund- und Zahnpflege- und -reinigungsmittel nach Anspruch 11, **dadurch gekennzeichnet, dass** es 0,01 bis 2,5 Gew.-%, vorzugsweise 0,05 bis 2,0 Gew.-%, besonders bevorzugt 0,1 bis 1,0 Gew.-%, weiter bevorzugt 0,11 bis 0,75 Gew.-% und insbesondere 0,12 bis 0,5 Gew.-% Calcium-Glycerophosphat enthält.

13. Mund und Zahnpflege- und -reinigungsmittel nach einem der Ansprüche 11 oder 12, **dadurch gekennzeichnet, dass** es 0,5 bis 9,5 Gew.-%, vorzugsweise 1 bis 9 Gew.-%, weiter bevorzugt 2 bis 8 Gew.-%, noch weiter bevorzugt 3 bis 7 Gew.-% und insbesondere 4 bis 6 Gew.-% Kaliumsalz(e) enthält.

14. Mund und Zahnpflege- und -reinigungsmittel nach einem der Ansprüche 11 bis 13, **dadurch gekennzeichnet, dass** es weniger als 60 Gew.-%, vorzugsweise weniger als 55 Gew.-%, besonders bevorzugt weniger als 50 Gew.-% und insbesondere weniger als 45 Gew.-% Wasser enthält.

15. Mund- und Zahnpflege- und -reinigungsmittel gemäß einem der Ansprüche 11 bis 14, **dadurch gekennzeichnet, dass** es zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthält.

## Claims

1. A coated fragrance and/or aroma particle, comprising a particle core and a shell surrounding this core, **characterized in that**
a) the particle core, based on the weight thereof, contains from 80 to 99.99 wt.% of silicate(s) and
b) the particle core, based on the weight thereof, contains from 0.01 to 20 wt.% of fragrance(s) and/or aromas and
c) the shell surrounding the core, based on the weight thereof, contains from 80 to 100 wt.% of water-soluble potassium silicates and/or sodium silicates,
the silicate or silicates of the particle core having particle sizes of between 10 and 1000 µm.

2. The particle according to claim 1, **characterized in that** the silicate or silicates have a weight-average particle size distribution in which > 70 wt.%, preferably > 80 wt.%, and in particular > 90 wt.%, of the particles have sizes of between 100 and 700 µm.

3. The particle according to one of claims 1 or 2, **characterized in that** the particle core additionally contains titanium dioxide.

4. The particle according to one of claims 1 to 3, **characterized in that** the particle core, based on the weight thereof, contains from 0.05 to 19 wt.%, preferably from 0.1 to 18 wt.%, particularly preferably from 0.5 to 17 wt.%, more preferably from 0.75 to 16 wt.%, and in particular from 1 to 15 wt.%, of at least one aroma from the group of peppermint, spearmint, menthol, citrus, lime, apple, orange, lemon, strawberry or aloe vera.

5. The particle according to one of claims 1 to 4, **characterized in that** the particle core, based on the weight thereof, additionally contains from 0.01 to 5 wt.%, preferably from 0.05 to 2.5 wt.%, and in particular from 0.1 to 1 wt.%, of surfactant(s).

6. The particle according to claim 5, **characterized in that** fatty alcohol sulfates, in particular sodium lauryl sulfate, and/or betaines, in particular cocoamidopropyl betaine, are used as surfactant(s).

7. The particle according to one of claims 1 to 6, **characterized in that** the shell contains water-soluble potassium silicates and/or sodium silicates ("water glasses"), which have a modulus of SiO₂ to MeO of M > 1, preferably of M > 2, and particularly preferably of M > 3.

8. The particle according to one of claims 1 to 7, **characterized in that** the shell makes up from 0.1 to 25 wt.%, preferably from 0.5 to 10 wt.%, and particularly preferably from 1 to 5 wt.%, of the coated particle.

9. The particle according to one of claims 1 to 8, **characterized in that** it contains at least one dye from the group of cationic (basic) dyes, preferably Basic Blue 6, C.I. no. 51175; Basic Blue 7, C.I. no. 42595; Basic Blue 9, C.I. no. 52015; Basic Blue 26, C.I. no. 44045; Basic Blue 41, C.I. no. 11154; Basic Blue 99, C.I. no. 56059; Basic Brown 4, C.I. no. 21010; Basic Brown 16, C.I. no. 12250; Basic Brown 17, C.I. no. 12251; Basic Green 1, C.I. no. 42040; Basic Orange 31; Basic Red 2, C.I. no. 50240; Basic Red 22, C.I. no. 11055; Basic Red 46; Basic Red 51; Basic Red 76, C.I. no. 12245; Basic Violet 1, C.I. no. 42535; Basic Violet 2; Basic Violet 3, C.I. no. 42555; Basic Violet 10, C.I. no. 45170; Basic Violet 14, C.I. no. 42510; Basic Yellow 57, C.I. no. 12719; Basic Yellow 87 and/or of anionic (acidic) dyes, and/or of non-ionic dyes, preferably Acid Black 1, C.I. no. 20470; Acid Black 52; Acid Blue 7; Acid Blue 9, C.I. no. 42090; Acid Blue 74, C.I. no. 73015; Acid Red 18, C.I. no. 16255; Acid Red 23; Acid Red 27, C.I. no. 16185; Acid Red 33; Acid Red 52; Acid Red 87, C.I. no. 45380; Acid Red 92, C.I. no. 45410; Acid Orange 3; Acid Orange 7; Acid Violet 43, C.I. no. 60730; Acid Yellow 1, C.I. no. 10316; Acid Yellow 10; Acid Yellow 23, C.I. no. 19140; Acid Yellow 3, C.I. no. 47005; Acid Yellow 36; D&C Brown no. 1, C.I. no. 20170 (Acid Orange 24); D&C Green no. 5, C.I. no. 61570 (Acid Green G); D&C Orange no. 4, C.I. no. 15510 (Acid Orange II); D&C Orange no. 10, C.I. no. 45425:1 (Solvent Red 73); D&C Orange no. 11, C.I. no. 45425 (Acid Red 95); D&C Red no. 21, C.I. no. 45380:2 (Solvent Red 43); D&C Red no. 27, C.I. no. 45410:1 (Solvent Red 48); D&C Red no. 33, C.I. no. 17200 (Acid Red 2A, Acid Red B); D&C Yellow no. 7, C.I. no. 45350:1 (Solvent Yellow 94); D&C Yellow no. 8, C.I. no. 45350 (Acid Yellow 73); FD&C Red no. 4, C.I. no. 14700 (Food Red 4); FD&C Yellow no. 6, C.I. no. 15985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; sodium picramate; 1,4-bis-(2'-hydroxyethyl)amino-2-nitro-p-phenylenediamine; HC Yellow 5; HC Blue 2; HC Blue 12; 4-amino-3-nitrophenol; HC Yellow 6; HC Yellow 12; 2-nitro-1-(2'hydroxyethyl)amino-4-methyl benzene; 2-nitro-4-amino-diphenylamine-2-carboxylic acid; 2-amino-6-chloro-4-nitrophenol; HC Red BN; 6-nitro-1,2,3,4-tetranitroquinoxaline; o-nitro-p-phenylenediamine; p-nitro-m-phenylendiamine; HC Red B 54; HC Red 10; HC Red 11; HC Red 13; 2-(2'-hydroxyethyl)amino-1-hydroxy-4,6-dinitrobenzene; 4-ethylamino-3-nitrobenzoic acid; 2-chloro-6-ethylamino-4-nitrophenol; 2-hydroxy-1,4-naphthoquinone; 1-propene-(4-amino-2-nitrophenyl)amine; isatin; N-methylisatin; HC Violet 1; HC Violet 2; 4-nitrophenyl aminoethylurea, in amounts of from 0.01 to 25 wt.%, preferably from 0.5 to 10 wt.%, in particular from 1 to 5 wt.%, in each case based on the particle including the coating.

10. A cosmetic hair and/or body cleansing agent, containing in a cosmetically acceptable carrier, based on the weight thereof,
a) from 0.5 to 20 wt.% of particles according to one of claims 1 to 9 and
b) from 5 to 50 wt.% of surfactant(s).

11. An oral and dental care and cleaning agent, containing, based on the weight thereof,
a) from 0.5 to 20 wt.% of particles according to one of claims 1 to 9 and
b) from 10 to 60 wt.%, preferably from 12.5 to 50 wt.%, particularly preferably from 15 to 45 wt.%, and in particular from 20 to 40 wt.%, of at least one polyhydric alcohol from the group of sorbitol, glycerol and 1,2-propyleneglycol.

12. The oral and dental care and cleaning agent according to claim 11, **characterized in that** it contains from 0.01 to 2.5 wt.%, preferably from 0.05 to 2.0 wt.%, particularly preferably from 0.1 to 1.0 wt.%, more preferably from 0.11 to 0.75 wt.%, and in particular from 0.12 to 0.5 wt.%, of calcium glycerophosphate.

13. The oral and dental care and cleaning agent according to one of claims 11 or 12, **characterized in that** it contains from 0.5 to 9.5 wt.%, preferably from 1 to 9 wt.%, more preferably from 2 to 8 wt.%, even more preferably from 3 to 7 wt.%, and in particular from 4 to 6 wt.%, of potassium salt(s).

14. The oral and dental care and cleaning agent according to one of claims 11 to 13, **characterized in that** it contains less than 60 wt.%, preferably less than 55 wt.%, particularly preferably less than 50 wt.%, and in particular less than 45 wt.%, of water.

15. The oral and dental care and cleaning agent according to one of claims 11 to 14, **characterized in that** it additionally contains anti-plaque active ingredients, preferably p-hydroxy benzoic acid methyl ester, p-hydroxy benzoic acid ethyl ester or p-hydroxy benzoic acid propyl ester, sodium sorbate, sodium benzoate, bromochlorophene, triclosan, hexetidines, phenyl salicylic acid ester, biguanides e.g. chlorhexidine, thymol, preferably in amounts of from 0.05 to 5 wt.%, preferably from 0.10 to 2.5 wt.%, and in particular from 0.30 to 1.5 wt.%, in each case based on the total agent.

## Revendications

1. Particule de parfum et/ou d'arôme revêtue, comprenant un noyau de particule et une enveloppe entourant ce noyau, **caractérisée en ce que**
a) le noyau de la particule - rapporté à son poids - contient 80 à 99,99 % en poids de silicate(s) et
b) le noyau de la particule contient - rapporté à son poids - contient 0,01 à 20 % en poids de parfum(s) et/ou d'arômes, et
c) l'enveloppe entourant le noyau - rapporté son poids - contient 80 à 100 % en poids de silicates de potassium et/ou de sodium hydrosolubles, dans lesquelles
le(s) silicate(s) du noyau de la particule présente(nt) des tailles de particules comprises entre 10 et 1 000 µm.

2. Particule selon la revendication 1, **caractérisée en ce que** le(s) silicate(s) présente(nt) une répartition granulométrique moyenne en poids dans laquelle plus de 70 % en poids, de préférence plus de 80 % en poids et en particulier plus de 90 % en poids des particules présentent des dimensions comprises entre 100 et 700 µm.

3. Particule selon l'une des revendications 1 ou 2, **caractérisée en ce que** le noyau des particules contient en outre du dioxyde de titane.

4. Particule selon l'une des revendications 1 à 3, **caractérisée en ce que** le noyau de la particule - rapporté à son poids - contient de 0,05 à 19 % en poids, de préférence de 0,1 à 18 % en poids, de manière particulièrement préférée de 0,5 à 17 % en poids, de manière davantage préférée de 0,75 à 16 % en poids et en particulier 1 à 15 % en poids d'au moins un arôme du groupe comprenant celui de la menthe poivrée, la menthe verte, le menthol, le citron vert, la limette, la pomme, l'orange, le citron, la fraise, l'aloe vera.

5. Particule selon l'une des revendications 1 à 4, **caractérisée en ce que** le noyau de la particule - rapporté à son poids - contient en plus de 0,01 à 5 % en poids, de préférence de 0,05 à 2,5 % en poids et en particulier de 0,1 à 1 % en poids en tensioactif(s).

6. Particule selon la revendication 5, **caractérisée en ce que** l'on utilise, comme tensioactif(s), les sulfates d'alcool gras, en particulier le laurylsulfate de sodium et/ou les bétaïnes, en particulier la bétaïne de cocoamidopropyle.

7. Particule selon l'une des revendications 1 à 6, **caractérisée en ce que** l'enveloppe contient des silicates de potassium et/ou de sodium hydrosolubles (« verres liquides ») ayant un module SiO₂ sur Me0 de M > 1, de préférence de M> 2 et de manière particulièrement préférée de M > 3.

8. Particule selon l'une des revendications 1 à 7, **caractérisée en ce que** l'enveloppe constitue de 0,1 à 25 % en poids, de préférence de 0,5 à 10 % en poids et de manière particulièrement préférée de 1 à 5 % en poids de la particule revêtue.

9. Particule selon l'une des revendications 1 à 8, **caractérisée en ce qu'**elle contient au moins un colorant issu du groupe des colorants cationiques (basiques), de préférence Basic Blue 6, C.I.-No. 51,175; Basic Blue 7, C.I.-No. 42,595; Basic Blue 9, C.I.-No. 52,015; Basic Blue 26, C.I.-No. 44,045 ; Basic Blue 41, C.I.-No. 11,154 ; Basic Blue 99, C.I.-No. 56,059 ; Basic Brown 4, C.I.-No. 21,010 ; Basic Brown 16, C.I.-No. 12,250 ; Basic Brown 17, C.I.-No. 12,251 ; Basic Green 1, C.I.-No. 42,040 ; Basic Orange 31 ; Basic Red 2, C.I.-No. 50,240 ; Basic Red 22, C.I.-No. 11,055 ; Basic Red 46 ; Basic Red 51 ; Basic Red 76, C.I.-No. 12,245 ; Basic Violet 1, C.I.-No. 42,535 ; Basic Violet 2 ; Basic Violet 3, C.I.-No. 42,555 ; Basic Violet 10, C.I.-No. 45,170 ; Basic Violet 14, C.I.-No. 42,510 ; Basic Yellow 57, C.I.-No. 12,719 ; Basic Yellow 87 et/ou des colorants anioniques (acides), et/ou des colorants non ioniques, de préférence Acid Black 1, C.I.-No. 20,470 ; Acid Black 52 ; Acid Blue 7 ; Acid Blue 9, C.I.-No. 42,090 ; Acid Blue 74, C.I.-No. 73,015, Acid Red 18, C.I.-No. 16,255 ; Acid Red 23 ; Acid Red 27, C.I.-No. 16,185 ; Acid Red 33 ; Acid Red 52 ; Acid Red 87, C.I.-No. 45,380 ; Acid Red 92, C.I.-No. 45,410 ; Acid Orange 3 ; Acid Orange 7 ; Acid Violet 43, C.I.-No. 60,730 ; Acid Yellow 1, C.I.-No. 10,316 ; Acid Yellow 10 ; Acid Yellow 23, C.I.-No. 19,140 ; Acid Yellow 3, C.I.-No. 47,005 ; Acid Yellow 36 ; D& C Brown No. 1, C.I.-No. 20,170 (Acid Orange 24) ; D&C Green No. 5, C.I.-No. 61,570 (Acid Green G) ; D&C Orange No. 4, C.I.-No. 15,510 (Acid Orange II) ; D&C Orange No. 10, C. I.-No. 45,425 : 1 (Solvent Red 73) ; D&C Orange No. 11, C.I.-No. 45,425 (Acid Red 95) ; D&C Red No. 21, C.I.-No. 45,380 : 2 (Solvent Red 43) ; D&C Red No. 27, C.I.-No. 45,410 : 1 (Solvent Red 48) ; D&C Red No. 33, C.I.-No. 17,200 (Acid Red 2A, Acid Red B) ; D&C Yellow No. 7, C. I.-No. 45,350 : 1 (Solvent Yellow 94) ; D&C Yellow No. 8, C.I.-No. 45,350 (Acid Yellow 73) ; FD& C Red No. 4, C.I.-No. 14,700 (Food Red 4) ; FD&C Yellow No. 6, C.I.-No. 15,985 (Food Yellow 3) ; Food Green 3 ; Pigment Red 57-1 ; Disperse Black 9 ; Disperse Blue 1 ; Disperse Blue 3 ; Disperse Violet 1 ; Disperse Violet 4 ; HC Orange 1 ; HC Red 1 ; HC Red 3 ; HC Red 13 ; HC Yellow 2 ; HC Yellow 4 ; Na-Pikramat ; 1,4-Bis-(2'-hydroxyéthyl)amino- 2-nitro-p- phénylènediamine ; HC Yellow 5 ; HC Blue 2 ; HC Blue 12 ; 4-amino-3-nitrophénol ; HC Yellow 6 ; HC Yellow 12 ; 2-Nitro-1-(2'hydroxyéthyl)amino-4-méthylbenzol ; acide 2-nitro-4-amino-diphénylamin- 2 -carboxylique ; 2-amino-6-chloro-4-nitrophénol ; HC Red BN ; 6-nitro-1,2,3,4-tétranitrochinoxaline ; o-nitro-p-phénylènediamine ; p-nitro-m-phénylènediamine ; HC Red B 54 ; HC Red 10 ; HC Red 11 ; HC Red 13 ; 2-(2'-hydroxyéthyl)amino-1-hydroxy-4,6-dinitrobenzol ; acide 4-éthylamino-3- nitrobenzoïque ; 2-chloro-6-éthylamino-4-nitrophénol ; 2-hydroxy-1,4-napthochinone ; 1-propène- (4-amino-2-nitrophényl)amine ; isatine ; N-méthylylisatine ; HC Violet 1 ; HC Violet 2 ; urée 4-nitrophényl-aminoéthyl dans des quantités allant de 0,01 à 25 % en poids, de préférence de 0,5 à 10 % en poids, en particulier de 1 à 5 % en poids, respectivement rapporté à la particule avec son revêtement.

10. Agent nettoyant cosmétique pour les cheveux et/ou le corps contenant dans un support cosmétiquement acceptable - rapporté son poids -
a) 0,5 à 20 % en poids d'une particule selon une des revendications 1 à 9 et
b) 5 à 50 % en poids de tensioactif(s).

11. Agent nettoyant et d'hygiène bucco-dentaire contenant - rapporté à son poids -
a) 0,5 à 20 % en poids d'une particule selon une des revendications 1 à 9 et
b) 10 à 60 % en poids, de préférence 12,5 à 50 % en poids, de manière particulièrement préférée 15 à 45 % en poids et en particulier 20 à 40 % en poids d'au moins un alcool plurivalent issu du groupe comprenant le sorbitol, la glycérine et le 1,2-propylène glycol.

12. Agent nettoyant et d'hygiène bucco-dentaire selon la revendication 11, **caractérisé en ce qu'**il contient de 0,01 à 2,5 % en poids, de préférence de 0,05 à 2,0 % en poids, de manière particulièrement préférée de 0,1 à 1,0 % en poids, de manière davantage préférée de 0,11 à 0,75 % en poids et en particulier de 0,12 à 0,5 % en poids de glycérophosphate de calcium.

13. Agent nettoyant et d'hygiène bucco-dentaire selon une des revendications 11 ou 12, **caractérisé en ce qu'**il contient de 0,5 à 9,5 % en poids, de préférence de 1 à 9 % en poids, de manière davantage préférée de 2 à 8 % en poids, de manière encore davantage préférée de 3 à 7 % en poids et en particulier de 4 à 6 % en poids de sel(s) de potassium.

14. Agent nettoyant et d'hygiène bucco-dentaire selon une des revendications 11 à 13, **caractérisé en ce qu'**il contient moins de 60 % en poids, de préférence moins de 55 % en poids, de manière particulièrement préférée moins de 50 % en poids et en particulier moins de 45 % en poids en eau.

15. Agent nettoyant et d'hygiène bucco-dentaire selon une des revendications 11 à 14, **caractérisé en ce qu'**il contient de plus des principes actifs anti-plaque, de préférence un ester méthylique, éthylique ou propylique d'acide p-hydroxybenzoïque, du sorbate de sodium, du benzoate de sodium, du chlorophène de brome, du triclosan, de l'hexétidine, de l'ester d'acide phényl-salicylique, des biguanides, par ex. de la chlorhexidine, du thymol, de préférence dans des quantités allant de 0,05 à 5 % en poids, de préférence de 0,10 à 2,5 % en poids et en particulier de 0,30 à 1,5 % en poids respectivement rapporté à l'ensemble de l'agent.
